# EUROPEAN PATENT SPECIFICATION

(11) **EP 1 467 762 B1**
(45) Date of publication and mention of the grant of the patent: **08.02.2006**
(21) Application number: 02804694.4
(22) Date of filing: 18.11.2002
(51) Int. Cl.: A61K 45/06, A61K 31/575, A61K 31/551, A61K 31/335, A61K 31/451, A61P 11/02

(54) **USE OF A H1 ANTAGONIST AND RIMEXOLONE AS A SAFE STEROID TO TREAT RHINITIS**
VERWENDUNG EINES H1-ANTAGONISTEN UND VON RIMEXOLON ALS SICHERES STEROID ZUR BEHANDLUNG VON RHINITIS
UTILISATION D'UN ANTAGONISTE H1 ET DU RIMEXOLONE COMME STEROIDE INOFFENSIF POUR TRAITER LA RHINITE

(30) Priority: 05.12.2001 US 337371 P
(43) Date of publication of application: 20.10.2004
(73) Proprietor: Alcon, Inc., 6331 Hünenberg (CH)
(72) Inventor: YANNI, John, M., Burleson, TX 76028 (US); GAMACHE, Daniel, A., Arlington, TX 76017 (US); MILLER, Steven T., Arlington, TX 76017 (US)
(74) Representative: Wain, Christopher Paul
(86) International application number: PCT/US2002/036915
(87) International publication number: WO 2003/049770

(56) References cited:
- WO-A-01/35963
- WO-A-97/01337
- DE-A- 19 947 234
- HOCHHAUS G ET AL: "BINDING AFFINITIES OF RIMEXOLONE ORG-6216 FLUNISOLIDE AND THEIR PUTATIVE METABOLITES FOR THE GLUCOCORTICOID RECEPTOR OF HUMAN SYNOVIAL TISSUE" AGENTS AND ACTIONS, vol. 30, no. 3-4, 1990, pages 377-380, XP009008536 ISSN: 0065-4299

## Description

The present invention is directed to the use of an H₁ antagonist/antiallergic in combination with a safe steroid to treat nasal conditions, specifically rhinitis.

### BACKGROUND OF THE INVENTION

Allergic rhinitis has historically been treated with a regimen of oral antihistamines and/or oral steroids. Systemic treatment typically requires higher concentrations of the drug compound to be administered to afford an effective concentration to reach the necessary treatment site. Antihistamine compounds are known to have central nervous system (CNS) activity which manifests itself in drowsiness. They may also have anticholinergic activity which manifests itself in the drying of mucus membranes. Steroid therapy whether dosed orally or intranasally can also produce significant systemic side effects, including adrenal insufficiency, cardio-vascular irregularities, and immunosuppression. Growth retardation is an especially important concern in allergic paediatric patients.

Intranasal combination therapy is known. For example, WO97/01337 discloses combinations of topical nasal antihistamines and topical nasal steroids for the treatment of rhinitis. It does not disclose the use of the safe steroids of the present invention. WO97/46243 discloses a nasal spray containing a steroid and an antihistamine. This publication does not disclose or suggest the use of a safe steroid. There are also intranasal products containing both a steroid and an antihistamine, among other active ingredients, (e.g., Cortinasal from Pharmacobel; Neovvine from Dupa; Nicorin from Rontag; Rinosular from SmithKline Beecham; Rinocusi from Cusi; and Comfonin from Meider.)

DE19947234 discloses the combination of loteprednol and azelastine for simultaneous, sequential or separate application in the treatment of allergies and respiratory tract diseases.

The use, as claimed herein, of an H₁ antagonist/antiallergic in combination with a safe steroid for treating rhinitis is not known.

### SUMMARY OF THE INVENTION

The present invention relates to intranasal compositions of combinations of H₁ antagonists/antiallergic and safe steroids to treat rhinitis. Methods for the use of the compositions in mammals are also contemplated.

### DESCRIPTION OF PREFERRED EMBODIMENTS

According to the present invention, there is provided the use of a composition comprising an H₁ antagonist/antiallergic selected from the group consisting of emedastine, loratadine, 5-[2-[4-bis(4-fluorophenyl)hydroxymethyl-1-piperidinyl]ethyl]-3-methyl]-2-oxazolidinone ethanedioate), desloratadine, azelastine, olopatadine, levocabastine, epinastine, and ketotifen, and a safe steroid which is rimexolone, for the manufacture of a medicament for the treatment of rhinitis in mammals.

The compositions of H₁ antagonists/antiallergics can be used for treating the sneezing and rhinorrhea associated with allergic rhinitis. The compositions also include a safe steroid, as used herein the term "safe steroid" means a steroid which treats eosinophil and neurotrophil associated inflammation with resultant congestion but has either a lack of systemic bioavailability or is rapidly deactivated after systemic absorption.

The H₁ antagonists/antiallergics which are useful according to the present invention include emedastine, loratadine, 5-[2-[4-bis(4-fluorophenyl)hydroxymethyl-1-piperidinyl]ethyl]-3-methyl]-2-oxazolidinone ethanedioate), desloratadine, azelastine, olopatadine, leveocabastine, epinastine, and ketotifen.

Safe steroids which can be used herein include rimexolone.

The H₁ antagonists/antiallergics and the safe steroid compound can be incorporated into various types of intranasal formulations for delivery to the nose. For example, intranasal formulations may contain preservatives, such as, benzalkonium chloride, EDTA, and tromethamine; viscosity modifiers, such as, hydroxy propyl methyl cellulose (HPMC) and related agents; toxicity adjusting agents, for example, sodium chloride (NaCl); wetting agents/surfactants, such as, tyloxapol or Polysorbate 80; pH adjusters; and water.

The compounds are preferably formulated as intranasal suspensions or solutions, with a pH of about 6.0 to 8.0. The H₁ antagonists/antiallergics will normally be contained in these formulations in an amount 0.01% to 0.5% by weight, but preferably in an amount of 0.02% to 0.1 % by weight. The safe steroids will normally be contained in those formulations in an amount 0.05% to 1.5% by weight, but preferably in an amount of 0.1% to 1.0% by weight. Thus, for intranasal presentation 1 to 2 sprays of these formulations would be delivered to the nostrils up to 2 times per day according to the routine discretion of a skilled clinician

The preferred compositions of the present invention includes olopatadine (0.1%) with rimexolone (0.1%) and emedastine 0.05% with rimexolone (0.1%).

The following example is illustrative of a composition of the present invention, but is in no way limiting.

### EXAMPLE

| **Ingredient** | **Weight %** |
|---|---|
| Emedastine | 0.05% |
| Rimexolone | 0.1 % |
| Benzalkonium chloride | 0.01% |
| Tromethamine | 0.5% |
| Disodium EDTA | 0.01% |
| Sodium Chloride (Adjust isotonicity to 280mOsmols/kg) | 0.6 to 0.8% |
| HPMC | 0.1 to 0.5% |
| Tyloxapol | 0.05% |
| NaOH and/or HCl | QS to pH 7.4 |
| Purified water | QS to 100% |

## Claims

1. Use of a composition comprising an H₁ antagonist/antiallergic selected from the group consisting of emedastine, loratadine, 5-[2-[4-bis(4-fluorophenyl)hydroxymethyl-1-piperidinyl]ethyl]-3-methyl]-2-oxazolidinone ethanedioate), desloratadine, azelastine, olopatadine, levocabastine, epinastine, and ketotifen, and a safe steroid which is rimexolone, for the manufacture of a medicament for the treatment of rhinitis in mammals.

2. The use according to claim 1 wherein the composition comprises an antagonist/antiallergic selected from the group consisting of emedastine, olopatadine, and desloratadine.

3. The use according to claim 1 wherein the composition comprises emedastine and rimexolone.

4. The use according to claim 1 wherein the composition comprises olopatadine and rimexolone.

5. The use according to claim 1 wherein the composition comprises desloratadine and rimexolone.

## Revendications

1. Utilisation d'une composition comprenant un antagoniste de H₁/antiallergique choisi dans le groupe consistant en l'emedastine, la loratadine, l'éthanedioate de 5-[2-[4-bis(4-fluorophényl)hydroxyméthyl-1-pipéridinyl]ethyl]-3-méthyl]-2-oxazolidinone), la desloratadine, l'azelastine, l'olopatadine, la levocabastine, l'épinastine et le kétotifen, et un stéroïde inoffensif qui est la rimexolone, pour la fabrication d'un médicament pour le traitement de la rhinite chez les mammifères.

2. Utilisation selon la revendication 1 où la composition comprend un antagoniste/antiallergique choisi dans le groupe consistant en l'emedastine, l'olopatadine et la desloratadine.

3. Utilisation selon la revendication 1 où la composition comprend de l'emedastine et de la rimexolone.

4. Utilisation selon la revendication 1 où la composition comprend de l'olopatadine et de la rimexolone.

5. Utilisation selon la revendication 1 où la composition comprend de la desloratadine et de la rimexolone.

## Patentansprüche

1. Verwendung einer Zusammensetzung, die eine H1-antagonistische / antiallergene Verbindung, die aus der Gruppe ausgewählt ist, die aus Emedastin, Loratadin, 5-[2-[4-Bis(4-fluorphenyl)hydroxymethyl-1-piperidinyl]-ethyl]-3-methyl]-2-oxazolidinonethandioat), Desloratadin, Azelastin, Olopatadin, Levocabastin, Epinastin und Ketotifen besteht, und ein sicheres Steroid umfasst, das Rimexolon ist, zur Herstellung eines Medikaments zur Behandlung von Rhinitis in Säugetieren.

2. Die Verwendung gemäß Anspruch 1, worin die Zusammensetzung eine antagonistische / antiallergene Verbindung umfasst, die aus der Gruppe ausgewählt ist, die aus Emedastin, Olopatadin und Desloratadin besteht.

3. Die Verwendung gemäß Anspruch 1, worin die Zusammensetzung Emedastin und Rimexolon umfasst.

4. Die Verwendung gemäß Anspruch 1, worin die Zusammensetzung Olopatadin und Rimexolon umfasst.

5. Die Verwendung gemäß Anspruch 1, worin die Zusammensetzung Desloratadin und Rimexolon umfasst.
